# EUROPÄISCHE PATENTSCHRIFT

(11) **EP 1 893 590 B1**
(45) Veröffentlichungstag und Bekanntmachung des Hinweises auf die Patenterteilung: **15.08.2012**
(21) Anmeldenummer: 06753939.5
(22) Anmeldetag: 27.05.2006
(51) Int. Cl.: C07D 263/58, A61K 31/423

(54) **Benzooxazol-2-on-Derivate als Inhibitoren von Lipasen und Phospholipasen**
Benzooxazole-2-on derivatives as lipase and phospholipase inhibitors
Dérivés de benzooxazol-2-one servant d'inhibiteurs de lipases et de phospholipases

(30) Priorität: 09.06.2005 DE 102005026808
(43) Veröffentlichungstag der Anmeldung: 05.03.2008
(60) Teilanmeldung: 10186482.5
(73) Patentinhaber: SANOFI, 75008 Paris (FR)
(72) Erfinder: ZOLLER, Gerhard, 65926 Frankfurt am Main (DE); PETRY, Stefan, 65926 Frankfurt am Main (DE); MÜLLER, Günter, 65926 Frankfurt am Main (DE); HEUER, Hubert, 65926 Frankfurt am Main (DE); TENNAGELS, Norbert, 65926 Frankfurt am Main (DE)
(74) Vertreter: Löwrick, Oliver
(86) Internationale Anmeldenummer: PCT/EP2006/005097
(87) Internationale Veröffentlichungsnummer: WO 2006/131233

(56) Entgegenhaltungen:
- EP-A- 0 463 944
- EP-A- 0 768 305
- WO-A-01/57002
- WO-A-2004/035550
- WO-A-2004/094393
- WO-A-2004/094394
- FR-A- 1 469 297
- RONALD J. NACHMAN: "3-(1-Imidazoyl)-6-methoxy-2-benzoxalinone ." JOURNAL OF HETEROCYCLIC CHEMISTRY, Bd. 22, 1985, Seiten 279-280, XP002392653 USPROVO, UT
- DATABASE CA [Online] CHEMICAL ABSTRACTS SERVICE, COLUMBUS, OHIO, US; AYUPOVA, A. T. ET AL: "Synthesis of some 3-acylbenzoxazolinones" XP002392688 gefunden im STN Database accession no. 2000:632971 & O'ZBEKISTON KIMYO JURNALI , (2), 30-33 CODEN: OKJZA6; ISSN: 0042-1707, 2000,
- DATABASE CA [Online] CHEMICAL ABSTRACTS SERVICE, COLUMBUS, OHIO, US; ZINNER, HELMUT ET AL: "Benzazoles. IV. Mannich bases of benzoxazolone" XP002392689 gefunden im STN Database accession no. 1957:39211 & CHEMISCHE BERICHTE , 89, 2131-6 CODEN: CHBEAM; ISSN: 0009-2940, 1956,

## Beschreibung

Die vorliegende Erfindung betrifft Benzooxazol-2-on-derivate der allgemeinen Formel I, deren pharmazeutisch anwendbare Salze und deren Verwendung als Arzneistoffe. Strukturähnliche Benzooxazolonderivate sind in DE 3102907, FR1469297 beschrieben. Diese werden aber als Pestizide und Fungizide verwendet. Strukturähnliche Benzooxazolonderivate mit pharmakologischer Wirkung sind in der WO01/57002 (als hypoglykämisches und hypolipidemisches Mittel), EP0768305 (als Endotholin Antagonist gegen Bluthochdruck), EP0463944 (als Mittel gegen Herz/Kreislauferkrankungen) beschrieben.

Verbindungen mit hemmender Wirkung auf die hormonsensitive Lipase sind im Stand der Technik beispielsweise in der WO20041035550, WO20051073199 oder WO03/051842 beschrieben.

Verbindungen mit hemmender Wirkung auf die endotheliale Lipase sind im Stand der Technik beispielsweise in der WO20041094394, WO2004/094393 beschrieben. Aufgabe der vorliegenden Erfindung ist die Bereitstellung von alternativen Verbindungen, die eine Hemmung der hormonsensitiven Lipase oder der endothelialen Lipase bewirken.

Gegenstand der Erfindung sind Benzooxazot-2-on-derivate der allgemeinen Formel I 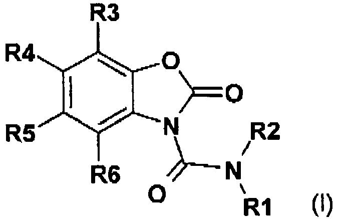 wobei bedeuten:
- R1: (C₅-C₁₆)-Alkyl, (C₁-C₄)-Alkylen-(C₆-C₁₀)-Aryl, Aryl durch Halogen, (C₁-C₆)-Alkyl, (C₁-C₃)-Alkyloxy, Hydroxy, (C₁-C₆)-Alkylmercapto, Amino, (C₁-C₆)-Alkylamino, di-(C₂-C₁₂)-Alkylamino, mono-(C₁-C₆)-Alkylaminocarbonyl, di-(C₂-C₈)-Alkylaminocarbonyl, (C₁-C₆)-Alkoxycarbonyl, (C₁-C₆)-Alkylcarbonyl, Cyano, Trifluormethyl, Trifluormethyloxy, (C₁-C₆)-Alkylsulfonyl, Aminosulfonyl ein oder mehrfach substituiert sein kann;
- R2: Wasserstoff;
- R3, R4, R5, R6: unabhängig voneinander Wasserstoff, Halogen, (C₁-C₆)-Alkyl, (C₁-C₃)-Alkyloxy-(C₁-C₃)-Alkylen, Hydroxy, (C₁-C₆)-Alkylmercapto, Amino, (C₁-C₆)-Alkylamino, di-(C₂-C₁₂)-A)kylamino, (C₁-C₆)-Alkylcarbonyl, COOR7, Trifluormethyl, (C₁-C₆)-Alkylsulfonyl, (C₁-C₆)-Alkylsulfinyl, Aminosulfonyl, Pentafluorsulfanyl, (C₆-C₁₀)-Aryl, (C₅-C₁₂-Heteroaryl, CO-NR9R10, O-CO-NR9R10, O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-NR9R10 oder unsubstituiertes oder ein oder mehrfach durch F substituiertes (C₁-C₆)-Alkyloxy; mit der Maßgabe, dass höchstens einer der Substituenten R3, R4, R5 oder R6 Halogen ist;
und mit der Maßgabe, dass höchstens einer der Substituenten R3, R4, R5 oder R6 (C₁-C₆)-Alkyl ist;
- R7: Wasserstoff, (C₁-C₆)-Alkyl, Benzyl;
- R9, R10: unabhängig voneinander Wasserstoff, (C₁-C₆)-Alkyl, -(C₆-C₁₀)-Aryl, (C₅-C₁₂)-Heteroaryl. (C₃-C₁₂)-Cycloalkyl, (C₁-C₄)-Alkylen-(C₆-C₁₀)-Aryl, (C₁-C₄)-Alkylen-(C₅-C₁₂)-Heteroaryl, (C₁-C₄)-Alkylen-(C₄-C₁₂)-Cycloalkyl, (C₈-C₁₄)-Bicyclus;
die tautomeren Formen der Verbindungen sowie deren physiologisch verträgliche Salze.

Bevorzugt sind Verbindungen der Formel I, worin
- R1: (C₆-C₁₂)-Alkyl, (C₁-C₃)-Alkylen-(C₆-C₁₀)-Aryl, wobei Aryl durch Halogen, (C₁-C₆)-Alkyl, (C₁-C₃)-Alkyloxy, Hydroxy, Amino, (C₁-C₆)-Alkylamino, Trifluormethyl ein oder mehrfach substituiert sein kann;
- R2: Wasserstoff;
- R3, R4, R5, R6: unabhängig voneinander Wasserstoff, (C₁-C₃)-Alkyloxy-(C₁-C₃)-Alkylen, Hydroxy, COOR7, (C₁-C₆)-Alkylsulfonyl, (C₁-C₆)-Alkylsulfinyl. Aminosulfonyl, Pentafluorsulfanyl, (C₆-C₁₀)-Aryl, (C₅-C₁₂)-Heteroaryl, (C₁-C₆)-Alkylcarbonyl, CO-NR9R10, O-CO-NR9R10, O-CD-(C₁-C₆)-Alkylen-CO-O-(C₁-C₆)-Alkyl, O-CO-(C₁-C₆)-Alkylen-CO-OH, O-CO-(C₁-C₆)-Alkylen-CO-NR9R10;
- R7: Wasserstoff, (C₁-C₈)-Alkyl, Benzyl;
- R9. R10: unabhängig voneinander Wasserstoff, (C₁-C₆)-Alkyl, (C₆-C₁₀)-Aryl, (C₁-C₄)-Alkylen-(C₆-C₁₀)-Aryl;
bedeuten.

Besonders bevorzugt sind Verbindungen der Formel **I,** worin
- R1: (C₆-C₁₂)-Alkyl, Benzyl, Benzyl durch Halogen, (C₁-C₆)-Alkyl, (C₁-C₃)-Alkyloxy, Hydroxy, Amino, (C₁-C₆)-Alkylamino, Trifluormethyl ein oder mehrfach substituiert sein kann;
- R2: Wasserstoff;
- R3, R4, R5, R6: unabhängig voneinander Wasserstoff, Hydroxy, COOR7, (C₁-C₆)-Alkylsulfonyl, Aminosulfonyl, Pentafluorsulfanyl, Phenyl, (C₁-C₆)-Alkylcarbonyl, CO-NR9R10, O-CO-NR9R10, O-CO-(C₁-C₆)-Alkylen -CO-O-(C₁-C₆)-Alkyl, O-CO-(C₁-C₆)-Alkylen-CO-OH, O-CO-(C₁-C₆)-Alkylen-CO-NR9R10;
- R7: Wasserstoff, (C₁-C₆)-Alkyl,
- R9, R10: unabhängig voneinander Wasserstoff, (C₁-C₆)-Alkyl;
bedeuten.

Eine weitere besonders bevorzugte Ausführungsform sind Verbindungen der Formel I, worin
- R3, R6: für Wasserstoff steht, R4 oder R5 nicht für Wasserstoff steht.

Ganz besonders bevorzugt sind Verbindungen der Formel I, worin
- R1: (C₆-C₁₂)-Alkyl, wobei, Benzyl durch Halogen, (C₁-C₆)-Alkyl, (C₁-C₃)-Alkyloxy, Hydroxy, Amino, (C₁-C₆)-Alkylamino, Trifluormethyl ein oder mehrfach substituiert sein kann;
- R2: Wasserstoff;
- R3: Wasserstoff;
- R4, R5: unabhängig voneinander Wasserstoff, Hydroxy, mono-(C₁-C₆)-Alkylaminocarbonyl, di-(C₂-C₈)-Alkylaminocarbonyl, COOR7, (C₁-C₆)-Alkylsulfonyl, Aminosulfonyl, Phenyl, (C₁-C₆)-Alkylcarbonyl, O-CO-NR9R10, O-CO-(C₁-C₆)-Alkylen -CO-O-(C₁-C₆)-Alkyl, O-CO-(C₁-C₆)-Alkylen-CO-OH, O-CO-(C₁-C₆)-Alkylen-CO-NR9R10;
- R6: Wasserstoff;
bedeuten.

Weiterhin ganz besonders bevorzugt sind die Verbindungen der Formel I, worin
- R1: (C₆-C₁₂)-Alkyl oder Benzyl, wobei Benzyl durch (C₁-C₆)-Alkyl ein oder mehrfach substituiert sein kann;
- R2: Wasserstoff;
- R3: Wasserstoff ;
- R4, R5: unabhängig voneinander Wasserstoff, Halogen oder (C₁-C₆)-Alkylcarbonyl;
- R6: Wasserstoff;
bedeuten.

Die Erfindung bezieht sich auf Verbindungen der Formel I, in Form ihrer Salze, Racemate, racemischen Mischungen und reinen Enantiomere sowie auf ihre Diastereomere und Mischungen davon.

Die Alkylreste in den Substituenten R1, R2, R3, R4, R5, R6, R7, R9 und R10 können sowohl geradkettig wie verzweigt sein. Halogen steht für Fluor, Chlor, Brom oder Iod, besonders für Fluor oder Chlor.

Unter Aryl wird ein aromatisches carbocyclisches mono- oder bicyclisches RingSystem verstanden, das 6 bis 10 Atome im Ring oder in den Ringen enthält. Heteroaryl ist ein mono- oder bicyclisches aromatisches Ringsystem mit 5 bis 12 Ringgliedern, worin mindestens ein Atom im Ringsystem ein Heteroatom aus der Reihe N, O und S ist.

Geeignete "Heteroaryl-Ringe" bzw. "Heteroaryl-Reste" sind beispielsweise Benzimidazolyl, Benzofuranyl, Benzothiophenyl, Benzoxazolyl, Benzthiazolyl, Benztriazolyl, Benztetrazolyl, Benzisoxazolyl, Benzisothiazolyl, Chinolinyl, Furyl, Furazanyl, Imidazolyl, 1H-Indazolyl, Indolyl, 1,2,3-Oxadiazolyl, 1,2,4-Oxadiazolyl, 1,2,5-Oxadiazolyl, 1,3,4-Oxadiazolyl, Pyrimidinyl, Pyrazinyl, Pyrazolyl, Pyridyl, Pyrrolyl, Thiazolyl, 1,2,3-Thiadiazolyl, 1,2,4-Thiadiazolyl, 1,2,5-Thiadiazolyl, 1,3,4-Thiadiazolyl, Thiophenyl.
Die Heteroaryl-Ringe bzw. Heteroaryl-Reste können ein oder mehrfach mit geeigneten Gruppen substituiert sein.

Ein Cycloalkylrest ist ein einen oder mehrere Ringe enthaltendes Ringssystem, welches gesättigt oder partiell ungesättigt (mit einer oder zwei Doppelbindungen) vorliegt, welches ausschließlich aus Kohlenstoffatomen aufgebaut ist.

Unter Bicyclus werden bicyclische Ringsysteme verstanden, welche gesättigt oder partiell ungesättigt vorliegen, welche außer Kohlenstoff noch ein oder mehrere Heteroatome, wie zum Beispiel Stickstoff, Sauerstoff oder Schwefel enthalten können. Ferner gehören auch Ringsysteme zu dieser Definition, die einen kondensierten Benzolkern enthalten. Beispielhaft seien der Tetrahydronaphthyl-, alpha- oder beta-Tetralon-, Indanyl- oder Indan-1-on-ylrest genannt. Bevorzugte Bicyclus-reste sind die der Formel Ic 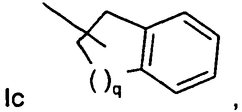 mit q =1 oder 2.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze) und Erdalkalisalze (wie Magnesiumund Calciumsalze) und Salze von Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen.

Nachfolgend beziehen sich alle Verweise auf "Verbindung(en) gemäß Formel I "auf Verbindung(en) der Formel I, wie vorstehend beschrieben, sowie ihre Salze, Solvate und physiologisch funktionelle Derivate wie hierin beschrieben.

### Verwendung

Die Verbindungen der allgemeinen Formel I,
die tautomeren Formen der Verbindungen sowie deren physiologisch verträgliche Salze, besitzen eine überraschende hemmende Wirkung an der Hormon Sensitiven Lipase, HSL, einem allosterischen Enzym in Adipozyten, das durch Insulin gehemmt wird und für den Abbau von Fetten in Fettzellen und damit für die Überführung von Fettbestandteilen in die Blutbahn verantwortlich ist. Eine Hemmung dieses Enzyms entspricht also einer insulinähnlichen Wirkung der erfindungsgemäßen Verbindungen, die letztlich zu einer Verminderung von freien Fettsäuren im Blut und von Blutzucker führt. Sie können also eingesetzt werden bei Entgleisungen des Stoffwechsels wie zum Beispiel beim nicht insulinabhängigen Diabetes Mellitus, beim diabetischen Syndrom und bei direkter Schädigung des Pankreas.

Die erfindungsgemäßen Verbindungen der allgemeinen Formel I, besonders diejenigen, in denen R2 Wasserstoff ist, können eine hemmende Wirkung auf die endotheliale Lipase (EL) besitzen. Das antiatheroskleotisch wirksame HDL ist das bevorzugte Substrat für EL. Eine Senkung des HDL-Spiegels führt zur Progression von Atherosklerose und ihrer Folgeerkrankungen wie Koronare Herzerkrankungen und begünstigt darüberhinaus die Entstehung des Metabolischen Syndroms und seiner Folgeerkrankung Diabetes. Eine Hemmung der EL sollte somit generell zur Vorbeugung von atherosklerotischen Erkrankungen führen und indirekt bei Personen mit erhöhtem Risiko für Diabetes die Erkrankungswahrscheinlichkeit verringern. Weiterhin wurde gefunden, dass die hemmende Wirkung der erfindungsgemäßen Verbindungen der allgemeinen Formel I selektiv gegenüber anderen Lipasen ist.
Die erfindungsgemäßen Verbindungen der Formel I können auch eine hemmende Wirkung auf die Triglyceridlipase aufweisen.

Besonders geeignet sind solche Verbindungen zur Behandlung und/oder Prävention von
1. Störungen des Fettsäurestoffwechsels und Glucoseverwertungsstörungen
2. Störungen der Insulin-Sensitivität von Muskel-, Fett- und Leberzellen (InsulinResistenz) - Metabolisches Syndrom
3. Diabetes mellitus, insbesondere Typ 2 Diabetes einschließlich der Verhinderung der damit verbundenen Folgeerkrankungen.
   Besondere Aspekte sind dabei die
   - Hyperglykämie,
   - Verbesserung der Insulinresistenz,
   - Verbesserung der Glucose-Toleranz,
   - Schutz der β-Zellen der Bauchspeicheldrüse
   - Verhinderung makro- und mikrovaskulärer Erkrankungen
4. Dyslipidämien und deren Folgen, wie z.B. Atherosklerose, koronare Herzkrankheit, zerebrovaskuläre Erkrankungen etc, insbesondere solche (aber nicht beschränkt auf), die durch einen oder mehrerer folgender Faktoren charakterisiert sind:
   - hohe Plasma-Triglycerid-, hohe postprandiale Plasma-Triglycerid-Konzentrationen
   - niedrige HDL-Cholesterin Konzentration
   - niedrige ApoA Lipoprotein-Konzentrationen
   - hohe LDL-Cholesterin Konzentrationen
   - kleine dichte LDL-Cholesterin Partikel
   - hohe ApoB Lipoprotein-Konzentrationen
5. Verschiedenen andere Zuständen, die mit dem Metabolischen Syndrom assoziert sein können, sind wie:
   - Adipositas (Fettsucht), einschließlich abdominale Adipositas
   - Thrombosen, Stadien von Hyperkoagulabilität und Thromboseneigung (arteriell und venös)
   - Hoher Blutdruck
   - Herzinsuffizienz, wie z.B. (aber nicht beschränkt auf) bei Zustand nach Myokardinfarkt, hypertensive Herzerkrankung oder Kardiomyopathie
6. weitere Krankheiten oder Zustände bei welchen zum Beispiel entzündliche Reaktionen oder die Zelldifferenzierung eine Rolle spielt sind:
   - Atherosklerose wie z.B. (aber nicht beschränkt auf) Koronarsklerose einschl. Angina pectoris oder Herzinfarkt, Hirnschlag
   - Vaskuläre Restenose oder Reverschluß
   - Chronisch entzündliche Darmerkrankungen, wie z.B. Morbus Crohn und Colitis ulcerosa
   - Pankreatitis
   - Andere entzündliche Zustände
   - Retinopathie
   - Fettzell-Tumore (adipose cell tumors)
   - Fettzell-Karzinome, wie z.B. Liposarkome
   - solide Tumoren und Neoplasien, wie z.B. (aber nicht beschränkt auf) Karzinome des Magen-Darm Traktes, der Leber, der Gallenwege und des Pankreas, endokrine Tumore, Karzinome der Lunge, der Niere und harnableitenden

Organe, des Genitaltraktes, Prostata-Karzinome etc.
- akute und chronische myeloprolifeative Erkrankungen und Lymphome
- Angiogenese
- Neurodegenerative Erkrankungen
- Alzheimersche Krankheit
- Multiple Sklerose
- Morbus Parkinson
- Erythemato-squamöse Dermatosen, wie z.B. Psoriasis (Schuppenflechte)
- Akne vulgaris
- Andere Hautkrankheiten und dermatologische Zustände, die durch PPAR moduliert werden
- Ekzeme und Neurodermitis
- Dermatitiden, wie z.B. seborrhoische Dermatitis oder Lichtdermatitis
- Keratitis und Keratosen, wie z.B. seborrhoische Keratosen, senile Keratosen, aktinische Keratose, photo-induzierte Keratosen oder Keratosis follicularis
- Keloide und Keloid-Prophylaxe
- Warzen, einschließlich Kondylomata oder Kondylomata acuminata
- Human papilloma viral (HPV) Infektionen, wie z.B. venerische Papillomata, virale Warzen, wie z.B. Molluscum contagiosum, Leukoplakie
- Papulöse Dermatosen, wie z.B. Lichen planus
- Hautkrebs, wie z.B. Basalzellkarzinome, Melanome oder kutane T-Zell Lymphome
- Lokalisierte, benigne epidermale Tumore, wie z.B. Keratoderma, epidermale Naevi
- Frostbeulen
- Hoher Blutdruck
- Syndrom X
- Syndrom der polyzystischen Ovarien (PCOS)
- Asthma
- Osteoarthritis
- Lupus erythematodes (LE) oder entzündliche rheumatische Erkrankungen, wie z.B. Rheumatoide Arthritis
- Vaskulitis
- Auszehrung (Kachexie)
- Gicht
- Ischämie/Reperfusions Syndrom
- Akutes respiratorisches Distress Syndrom (ARDS) ("Schocklunge")

Verbindungen, welche die endotheliale Lipase hemmen, sind besonders geeignet zur Behandlung und/oder Prävention von
1. Dyslipidämien und allgemeine Störungen des Fettstoffwechsels und deren Folgen, wie z.B. Atherosklerose, koronare Herzkrankheit, zerebrovaskuläre Erkrankungen etc, insbesondere solche (aber nicht beschränkt auf), die durch einen oder mehrerer folgender Faktoren charakterisiert sind:
   - hohe Plasma-Triglycerid-, hohe postprandiale Plasma-Triglycerid-Konzentrationen
   - niedrige HDL-Cholesterin Konzentration
   - niedrige ApoA Lipoprotein-Konzentrationen
   - hohe LDL-Cholesterin Konzentrationen
   - kleine dichte LDL-Cholesterin Partikel
   - hohe ApoB Lipoprotein-Konzentrationen
2. Verschiedenen andere Zuständen, die mit dem Metabolischen Syndrom assoziert sein können, sind wie:
   - Adipositas (Fettsucht), einschließlich abdominale Adipositas
   - Thrombosen, Stadien von Hyperkoagulabilität und Thromboseneigung (arteriell und venös)
   - Hoher Blutdruck
   - Herzinsuffizienz, wie z.B. (aber nicht beschränkt auf) bei Zustand nach Myokardinfarkt, hypertensive Herzerkrankung oder Kardiomyopathie
   - Diabetes mellitus, insbesondere Typ 2 Diabetes einschließlich der Verhinderung der damit verbundenen Folgeerkrankungen (Hyperglykämie, Glucoseintoleranz, Verlust der β-Zellen der Bauchspeicheldrüse, makro- und mikrovaskulärer Erkrankungen
3. weitere Krankheiten oder Zustände bei welchen zum Beispiel entzündliche Reaktionen oder die Zelldifferenzierung eine Rolle spielt sind:
   - Atherosklerose wie z.B. (aber nicht beschränkt auf) Koronarsklerose einschl. Angina pectoris oder Herzinfarkt, Hirnschlag
   - Vaskuläre Restenose oder Reverschluß
   - Chronisch entzündliche Darmerkrankungen, wie z.B. Morbus Crohn und Colitis ulcerosa
   - Pankreatitis
   - Andere entzündliche Zustände
   - Retinopathie
   - Fettzell-Tumore (adipose cell tumors)
   - Fettzell-Karzinome, wie z.B. Liposarkome
   - solide Tumoren und Neoplasien, wie z.B. (aber nicht beschränkt auf) Karzinome des Magen-Darm Traktes, der Leber, der Gallenwege und des Pankreas, endokrine Tumore, Karzinome der Lunge, der Niere und harnableitenden Organe, des Genitaltraktes, Prostata-Karzinome etc.
   - akute und chronische myeloprolifeative Erkrankungen und Lymphome
   - Angiogenese
   - Neurodegenerative Erkrankungen
   - Alzheimersche Krankheit
   - Multiple Sklerose
   - Morbus Parkinson
   - Erythemato-squamöse Dermatosen, wie z.B. Psoriasis (Schuppenflechte)
   - Akne vulgaris
   - Andere Hautkrankheiten und dermatologische Zustände, die durch PPAR moduliert werden
   - Ekzeme und Neurodermitis
   - Dermatitiden, wie z.B. seborrhoische Dermatitis oder Lichtdermatitis
   - Keratitis und Keratosen, wie z.B. seborrhoische Keratosen, senile Keratosen, aktinische Keratose, photo-induzierte Keratosen oder Keratosis follicularis
   - Keloide und Keloid-Prophylaxe
   - Warzen, einschließlich Kondylomata oder Kondylomata acuminata
   - Human papilloma viral (HPV) Infektionen, wie z.B. venerische Papillomata, virale Warzen, wie z.B. Molluscum contagiosum, Leukoplakie
   - Papulöse Dermatosen, wie z.B. Lichen planus
   - Hautkrebs, wie z.B. Basalzellkarzinome, Melanome oder kutane T-Zell Lymphome
   - Lokalisierte, benigne epidermale Tumore, wie z.B. Keratoderma, epidermale Naevi
   - Frostbeulen
   - Hoher Blutdruck
   - Syndrom X
   - Syndrom der polyzystischen Ovarien (PCOS)
   - Asthma
   - Osteoarthritis
   - Lupus erythematodes (LE) oder entzündliche rheumatische Erkrankungen, wie z.B. Rheumatoide Arthritis
   - Vaskulitis
   - Auszehrung (Kachexie)
   - Gicht
   - lschämie/Reperfusions Syndrom
   - Akutes respiratorisches Distress Syndrom (ARDS) ("Schocklunge")

### Galenik

Die Menge einer erfindungsgemäßen Verbindung, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg bis 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 0.05 bis 1000 mg, typischerweise von 0,5 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer erfindungsgemäßer Verbindungen. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Zubereitungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mitteln in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, welche die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im Algemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Die Verbindungen der Formel I zeichnen sich durch günstige Wirkungen auf Stoffwechselstörungen aus. Sie beeinflussen den Fett- und Zuckerstoffwechsel positiv, sie senken insbesondere den Triglyceridspiegel und sind zur Prävention und Behandlung von Typ II Diabetes und Arteriosklerose sowie deren vielfältigen Folgeerkrankungen geeignet.

Die Verbindungen der Formel I mit R2 Wasserstoff zeichnen sich durch günstige Wirkungen auf Lipidstoffwechselstörungen aus. Sie beeinflussen das Verhältnis von HDL zu LDL positiv und erhöhen besonders die HDL-Spiegel und sind zur Prävention und Behandlung von Dyslipidämien und Metabolischem Syndrom sowie deren vielfältigen Folgeerkrankungen wie Atherosklerose, koronare Herzerkrankungen, Herzinsuffizienz, Adipositas und Diabetes geeignet.

### Kombinationen mit anderen Medikamenten

Die erfindungsgemäßen Verbindungen können allein oder in Kombination mit einer oder mehreren weiteren pharmakologisch wirksamen Substanzen verabreicht werden, die beispielsweise günstige Wirkungen auf Stoffwechselstörungen oder damit häufig assoziierte Erkrankungen haben. Solche Medikamente sind zum Beispiel
1. Blutzuckersenkende Medikamente, Antidiabetika,
2. Wirkstoffe zur Behandlung von Dyslipidemien,
3. Antiatherosklerotische Medikamente,
4. Antiadiposita,
5. Antiinflammatorische Wirkstoffe
6. Wirkstoffe zur Behandlung von malignen Tumoren
7. Antithrombotische Wirkstoffe
8. Wirkstoffe zur Behandlung von Bluthochdruck
9. Wirkstoffe zur Behandlung von Herzinsuffizienz sowie
10. Wirkstoffe zur Behandlung und/oder Prävention von Komplikationen, die von Diabetes verursacht werden oder mit Diabetes assoziiert sind.

Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen.

Beispielhaft seien genannt:

### Antidiabetika

Geeignete Antidiabetika sind z.B. die in der Roten Liste 2001, Kapitel 12 oder USP Dictionary of USAN and International Drug Names, US Pharmacopeia, Rockville 2003, offenbart. Antidiabetika umfassen alle Insuline und Insulinderivate, wie z.B. Lantus^{®} (siehe www.lantus.com) oder Apidra^{®}, sowie andere schnell wirkende Insuline (siehe US 6,221,633), GLP-1-Rezeptor Modulatoren, wie in WO 01/04146 beschrieben, oder auch wie z.B. diejenigen, die in WO 98/08871 von Novo Nordisk A/S offenbart wurden. Die oral wirksamen hypoglykämischen Wirkstoffe umfassen vorzugsweise die auf den ATP-abhängigen Kaliumkanal der Betazellen wirken Sulphonylharnstoffe, (z.B. in WO 97/26265 und WO 99/03861 offenbart), Biguanide, Meglitinide, Glukagon-Antagonisten, orale GLP-1-Agonisten, DPP-IV Inhibitoren, Insulin-Sensitizer, z.B. PPAR - und PXR-Modulatoren und Wirkstoffe wie z.B. Oxadiazolidindione, Thiazolidindione, Inhibitoren von Leberenzymen, die an der Stimulation der Glukoneogenese und/oder Glykogenolyse beteiligt sind, Modulatoren der Glukoseaufnahme wie z.B. Glukosidase-Inhibitoren, den Fettstoffwechsel verändernde Verbindungen, die zur Veränderung der Lipidzusammensetzung des Blutes führen, Verbindungen, die die Nahrungsmitteleinnahme oder Nahrungsmittelaufnahme verringern.

Bei einer Ausführungsform der Erfindung werden die Verbindungen der Formel I in Kombination mit Insulin verabreicht.
Bei einer Ausführungsform der Erfindung werden die Verbindungen der Formel I in Kombination mit Substanzen, die Einfluss haben auf die hepatische Glukoseproduktion verabreicht, wie z.B. Glycogen Phosphorylase Inhibitoren (siehe: WO 01/94300, WO 02/096864, WO 03/084923, WO 03/084922, WO 03/104188)
Bei einer Ausführungsform werden die Verbindungen der Formel I in Kombination mit einem Wirkstoff verabreicht, der auf den ATP-abhängigen Kaliumkanal der Betazellen wirkt, wie z.B. Sulphonylharnstoffe (z.B. Tolbutamid, Glibenclamid, Glipizid, Glimepirid) oder Glinide (z.B. Repaglinid).
Bei einer Ausführungsform werden die Verbindungen der Formel I in Kombination mit einem Biguanid, wie z.B. Metformin, verabreicht.

Bei einer Ausführungsform werden die Verbindungen der Formel I in Kombination mit einem PPARgamma Agonist oder Thiazolidindion, wie z.B., Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.
Bei einer Ausführungsform werden die Verbindungen der Formel I in Kombination mit einem DPPIV Inhibitor, wie z.B. in WO98/19998, WO99/61431, WO99/67278, WO99/67279, WO01/72290, WO 02/38541, WO03/040174 beschrieben, insbesondere P 93/01 (1-Cyclopentyl-3-methyl-1-oxo-2-pentanammonium chlorid), P-31/98, LAF237 (1-[2-[3-Hydroxyadamant-1-ylamino)acetyl]pyrrolidin-2-(S)-carbonitril), TS021 ((2S, 4S)-4-Fluoro-1-[[(2-hydroxy-1,1-dimethylethyl)amino]-acetyl]-pyrrolidin-2-carbonitril monobenzenesulfonat)

Bei einer Aufführungsform werden die Verbindungen der Formel I in Kombination mit Verbindungen mit inhibitorischer Wirkung auf SGLT-1 und/oder 2, wie z.B. in WO2004/007517, WO2004/052902, WO2004/052903 und WO2005/121161 direkt oder indirekt offenbart, verabreicht.

Bei einer Ausführungsform werden die Verbindungen der Formel I in Kombination mit einem α-Glukosidase-Inhibitor, wie z.B. Miglitol oder Acarbose, verabreicht.
Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

### Lipidmodulatoren

Bei einer Ausführungsform der Erfindung werden die Verbindungen der Formel I in Kombination mit einem HMGCoA-Reduktase Inhibitor wie Lovastatin , Fluvastatin, Pravastatin, Simvastatin, Ivastatin, Itavastatin, Atorvastatin, Rosuvastatin verabreicht.

Bei einer Ausführungsform der Erfindung werden die Verbindungen der Formel I in Kombination mit einem Gallensäureresorptionsinhibitor verabreicht (siehe z.B. US 6,245,744, US 6,221,897, US 6,277,831, EP 0683 773, EP 0683 774).

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem Cholesterinresorptionsinhibitor, wie z.B. in WO 02/50027 beschrieben, oder Ezetimibe, Tiqueside, Pamaqueside verabreicht.

Bei einer Ausführungsform der Erfindung werden die Verbindungen der Formel I in Kombination mit einem LDL-Rezeptorinduktor (siehe z.B. US 6,342,512) verabreicht.

Bei einer Ausführungsform werden die Verbindungen der Formel I in Kombination mit Ballaststoffen, vorzugsweise unlöslichen Ballaststoffen (siehe z.B. Carob/Caromax^{®} (Zunft H J; et al., Carob pulp preparation for treatment of hypercholesterolemia, ADVANCES IN THERAPY (2001 Sep-Oct), 18(5), 230-6)); Caromax ist ein Carob enthaltendes Produkt der Fa. Nutrinova, Nutrition Specialties & Food Ingredients GmbH, Industriepark Höchst, 65926 Frankfurt / Main) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Bei einer Ausführungsform der Erfindung werden die Verbindungen der Formel I in Kombination mit einem PPARalpha Agonist verabreicht.

Bei einer Ausführungsform der Erfindung werden die Verbindungen der Formel I in Kombination mit einem gemischten PPAR alpha/gamma Agonisten, wie z.B. AZ 242 (Tesaglitazar, (S)-3-(4-[2-(4-Methansulfonyloxyphenyl)ethoxy]phenyl)-2-ethoxypropionsäure), BMS 298585 (N-[(4-Methoxyphenoxy)carbonyl]-N-[[4-[2-(5-methyl-2-phenyl-4-oxazolyl)ethoxy]phenyl]methyl]glycin) oder wie in WO 99/62872, WO 99/62871, WO 01/40171, WO 01/40169, WO 96/38428, WO 01/81327, WO 01/21602, WO 03/020269, WO 00/64888 oder WO 00/64876 beschrieben, verabreicht.

Bei einer Ausführungsform der Erfindung werden die Verbindungen der Formel I in Kombination mit einem Fibrat, wie z.B. Fenofibrat, Gemfibrozil, Clofibrat, Bezafibrat, verabreicht.

Bei einer Ausführungsform der Erfindung werden die Verbindungen der Formel I in Kombination mit Nicotinsäure bzw. Niacin verabreicht.

Bei einer Ausführungsform der Erfindung werden die Verbindungen der Formel I in Kombination mit einem CETP-Inhibitor, z.B. CP- 529, 414 (Torcetrapib), verabreicht.

Bei einer Ausführungsform der Erfindung werden die Verbindungen der Formel I in Kombination mit einem ACAT-Inhibitor verabreicht.

Bei einer Ausführungsform der Erfindung werden die Verbindungen der Formel lin Kombination mit einem MTP-Inhibitor, wie z.B. lmplitapide, verabreicht.

Bei einer Ausführungsform der Erfindung werden die Verbindungen der Formel I in Kombination mit einem Antioxidanz verabreicht.

Bei einer Ausführungsform der Erfindung werden die Verbindungen der Formel I in Kombination mit einem Lipoprotein-Lipase Inhibitor, verabreicht.

Bei einer Ausführungsform der Erfindung werden die Verbindungen der Formel I in Kombination mit einem ATP-Citrat-Lyase Inhibitor verabreicht.

Bei einer Ausführungsform der Erfindung werden die Verbindungen der Formel I in Kombination mit einem Squalen Synthetase Inhibitor verabreicht.

Bei einer Ausführungsform der Erfindung werden die Verbindungen der Formel I in Kombination mit einem Lipoprotein(a) Antagonist verabreicht.

### Antiobesita

Bei einer Ausführungsform der Erfindung werden die Verbindungen der Formel I in Kombination mit einem Lipase Inhibitor, wie z.B. Orlistat, verabreicht.

Bei einer Ausführungsform ist der weitere Wirkstoff Fenfluramin oder Dexfenfluramin. Bei noch einer Ausführungsform ist der weitere Wirkstoff Sibutramin.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.: Hormone and Metabolic Research (2001), 33(9), 554-558), NPY-Antagonisten z.B. Naphthalin-1-sulfonsäure {4-[(4-amino-chinazolin-2-ylamino)-methyl]-cyclohexylmethyl}-amid; hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)), Orexin-Antagonisten (z.B. 1-(2-Methyl-benzoxazol-6-yl)-3-[1,5]naphthyridin-4-yl-harnstoff; hydrochloride (SB-334867-A)), H3-Agonisten (3-Cyclohexyl-1-(4,4-dimethyl-1,4,6,7-tetrahydro-imidazo[4,5-c]pyridin-5-yl)-propan-1- on Oxalsäuresalz (WO 00/63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B. 1-(4-Chloro-3-methanesulfonylmethyl-phenyl)-2-[2-(2,3-dimethyl-1 H-indol-6-yloxy)-ethylamino]-ethanol; hydrochloride (WO 01/83451)), MSH (Melanocyt-stimulierendes Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7-dimethyl-indol-1-yl}-acetic acid Trifluoressigsäuresalz (WO 99/15525)); Serotonin-Wiederaufnahme-Inhibitoren (z.B. Dexfenfluramine), gemischte Serotonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxy-1-(2-diisopropylamino-ethylcarbamoyl)-3,4-dihydro-1H-isoquinoline-2-carboxylic acid tert-butyl ester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881),
DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR-β-Agonisten verabreicht.

Bei noch einer Ausführungsform ist der weitere Wirkstoff ein Cannabinoid Rezeptor 1 Antagonist (wie z.B. Rimonabant, SR147778 oder solche wie sie in z. B. EP 0656354, WO 00/15609, WO 02/076949, WO2005080345, WO2005080328, WO2005/080343, WO2005/075450, WO2005/080357, W02001/70700, WO2003/026647-48, WO2003/02776, WO2003/040107, WO2003/007887, WO2003/027069, US6,509,367, WO2001/32663, WO2003/086288, WO2003/087037, WO2004/048317, WO2004/058145, WO2003/084930, WO2003/084943, WO2004/058744, WO2004/013120, WO2004/029204, W02004/035566, WO2004/058249, WO2004/058255, WO2004/058727, W02004/069838, US2004/0214837, US2004/0214855, US2004/0214856, WO2004/096209, WO2004/096763, WO2004/096794, WO2005/000809, WO2004/099157, US2004/0266845, WO2004/110453, WO2004/108728, WO2004/000817, WO2005/000820, US2005/0009870, WO2005/00974, W02004/111033-34, WO2004/11038-39, WO2005/016286, WO2005/007111, WO2005/007628, US2005/0054679, WO2005/027837, WO2005/028456, W02005/063761-62, WO2005/061509, WO2005/077897 beschrieben sind);

Bei einer Ausführungsform der Erfindung ist der weitere Wirkstoff Leptin.

Bei einer Ausführungsform ist der weitere Wirkstoff Dexamphetamin, Amphetamin, Mazindol oder Phentermin.

Bei einer Ausführungsform werden die Verbindungen der Formel I in Kombination mit Medikamenten mit Wirkungen auf das Herz-Kreislauf- und das Blutgefäß-System, verabreicht, wie z.B. ACE-Hemmer (z.B. Ramipril), Medikamente, die auf das Angiotensin-Renin-System wirken, Calcium-Antagonisten, Beta-Blocker etc.

Bei einer Ausführungsform werden die Verbindungen der Formel I in Kombination mit anti-entzündlich wirkenden Medikamenten verabreicht.

Bei einer Ausführungsform werden die Verbindungen der Formel I in Kombination mit Medikamenten, die zur Krebstherapie und Krebsprävention eingesetzt werden, verabreicht.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

Die Wirksamkeit der erfindungsgemäßen Verbindungen der Formeln I wurde an folgenden Enzymtestsystemen geprüft:

### 1. Test auf Hemmung der HSL

### 1.1. Präparation der partiell gereinigten HSL:

Isolierte Rattenfettzellen werden aus Nebenhodenfettgewebe von nicht-behandelten männlichen Ratten (Wistar, 220-250 g) durch Kollagenasebehandlung gemäß publizierter Verfahren gewonnen (z.B. S. Nilsson et al., Anal. Biochem. 158, 1986, 399 - 407; G. Fredrikson et al., J. Biol. Chem. 256, 1981, 6311 - 6320; H. Tornquist et al., J. Biol. Chem. 251, 1976, 813 - 819). Die Fettzellen aus 10 Ratten werden dreimal durch Flotation mit jeweils 50 ml Homogenisationspuffer (25 ml Tris/HCl, pH 7.4, 0.25 M Sucrose, 1 mM EDTA, 1 mM DTT, 10 µg/ml Leupeptin, 10 µglml Antipain, 20 µg/ml Pepstatin) gewaschen und schließlich in 10 ml Homogenisationspuffer aufgenommen. Die Fettzellen werden im Teflon-in-Glas Homogenisator (Braun-Melsungen) durch 10 Hübe bei 1500 rpm und 15°C homogenisiert. Das Homogenisat wird zentrifugiert (Sorvall SM24-Röhrchen, 5000 rpm, 10 min, 4°C). Der Unterstand zwischen der oben liegenden Fettschicht und dem Pellet wird abgenommen und die Zentrifugation wiederholt. Der daraus resultierende Unterstand wird erneut zentrifugiert (Sorvall SM24-Röhrchen, 20000 rpm, 45 min, 4°C). Der Unterstand wird abgenommen und mit 1 g Heparin-Sepharose (Pharmacia-Biotech, CL-6B, 5 x gewaschen mit 25 mM Tris/HCl, pH 7.4, 150 mM NaCl) versetzt. Nach Inkubation für 60 min bei 4°C (in Intervallen von 15 min aufschütteln) wird der Ansatz zentrifugiert (Sorvall SM24-Röhrchen, 3000 rpm, 10 min, 4°C). Der Überstand wird durch Zugabe von Eisessig auf pH 5.2 gebracht und 30 min bei 4°C inkubiert. Die Präzipitate werden durch Zentrifugation (Sorvall SS34, 12000 rpm, 10 min, 4°C) gesammelt und in 2.5 ml 20 mM Tris/HCl, pH 7.0, 1 mM EDTA, 65 mM NaCl, 13 % sucrose, 1 mM DTT, 10 µg/ml Leupeptin/Pepstatin/Antipain suspendiert. Die Suspension wird über Nacht bei 4°C gegen 25 mM Tris/HCl, pH 7.4, 50 % Glyzerin, 1 mM DTT, 10 µg/ml Leupeptin, Pepstatin, Antipain dialysiert und dann auf eine Hydroxiapatit-Säule aufgetragen (0.1 g pro 1 ml Suspension, äquilibriert mit 10 mM Kaliumphosphat, pH 7.0, 30 % Glyzerin, 1 mM DTT). Die Säule wird mit vier Volumina Äquilibrierungspuffer bei einer Flußrate von 20 bis 30 ml/h gewaschen. Die HSL wird mit einem Volumen Äquilibrierungspuffer, der 0.5 M Kaliumphosphat enthält, eluiert, sodann dialysiert (s.o.) und 5- bis 10-fach konzentriert durch Ultrafiltration (Amicon Diaflo PM 10 Filter) bei 4°C. Die partiell gereinigte HSL kann 4 bis 6 Wochen bei -70°C aufbewahrt werden.

### 1.2. Assay auf HSL-Aktivität:

Für die Herstellung des Substrats werden 25-50 µCi [3H]Trioleoylglycerin (in Toluol), 6.8 µMol unmarkiertes Trioleoylglycerin und 0.6 mg Phospholipide (Phosphatidylcholin/Phosphatidylinositol 3:1 w/v) gemischt, über N2 getrocknet und dann in 2 ml 0.1 M KPi (pH 7.0) durch Ultraschallbehandlung (Branson 250, Mikrospitze, Einstellung 1-2, 2 x 1 min im 1-min Intervall) aufgenommen. Nach Zugabe von 1 ml KPi und erneuter Ultraschallbehandlung (4 x 30 sec auf Eis in 30-sec Intervallen) wird 1 ml 20% BSA (in KPi) hinzugefügt (Endkonzentration Trioleoylglyzerin 1.7 mM). Für die Reaktion werden 100 µl Substratlösung zu 100 µl HSL-Lösung (HSL präpariert wie oben, verdünnt in 20 mM KPi, pH 7.0, 1 mM EDTA, 1 mM DTT, 0.02% BSA, 20 µg/ml Pepstatin, 10 µg/ml Leupeptin) pipettiert und für 30 min bei 37°C inkubiert. Nach Zugabe von 3.25 ml Methanol/Chloroform/Heptan (10:9:7) und von 1.05 ml 0.1 M K2CO3, 0.1 M Borsäure (pH 10.5) wird gut gemischt und schließlich zentrifugiert (800 x g, 20 min). Nach Phasentrennung wird ein Äquivalent der oberen Phase (1 ml) abgenommen und die Radioaktivität durch Flüssigszintillationsmessung bestimmt.

### 1.3. Auswertung der HSL-Hemmwirkung:

Substanzen werden üblicherweise in vier unabhängigen Ansätzen geprüft. Die Hemmung der enzymatischen Aktivität der HSL durch eine Testsubstanz wird durch den Vergleich mit einer nicht-gehemmten Kontrollreaktion bestimmt. Die Berechnung des IC50-Wertes erfolgt über eine Hemmkurve mit mind. 10 Konzentrationen der Testsubstanz. Für die Analyse der Daten wird das Softwarepaket GRAPHIT, Elsevier-BIOSOFT, benutzt.

### 2. Test auf Hemmung der EL:

### 2.1. Präparation der EL

EL wird als sekretorisches Protein von rekombinanten Zell-Linien (CHO, HEK293) in hoher Konzentration in Zellkulturmedium abgegeben (konditioniertes Medium). Dieses wird nach Aufkonzentration als Enzymlösung eingesetzt.

### 2.2. Assay auf EL-Aktivität

Zur Charakterisierung der enzymatischen Aktivität von endothelialer Lipase und der Wirkung von Inhibitoren wird das Phospholipase-spezifische Substrat 1,2-bis-(4,4-difluoro-5,7-dimethyl-4-bora-3a,4a-diaza-s-indacene-3-undecanoyl)-sn-glycero-3-phosphocholine, (Hersteller Molecular Probes) verwendet. Durch Hydrolyse der A1 Esterbindung dieses Phospholipids durch das Enzym wird der Fluoreszenzfarbstoff Bodipy freigesetzt, der nach Trennung durch Dünnschichtchromatographie auf einer HPTLC-Platte (Kieselgel 60, Merck) oder direkt im Reaktionsgefäß durch Messen der Fluoreszenz nachgewiesen werden kann.
Zur Herstellung der Substratlösung werden 100 µg 1,2-bis-(4,4-difluoro-5,7-dimethyl-4-bora-3a,4a-diaza-s-indacene-3-undecanoyl)-sn-glycero-3-phospho-choline (Hersteller Molecular Probes), 2,4 mg Tripalmitin (Sigma) und 7,9 mg DOP - Cholin (1,2-Dioleoylsn-glycero-3-phosphocholin) in 393 µl Chloroform aufgenommen und im Anschluss 157 µl in ein frisches Reaktionsgefäß überführt. Nach Abdampfen des Lösungsmittels wird das Lipidgemisch in 4 ml 200 mM TRIS-HCl, 150 mM Natriumchlorid, pH = 7,4 durch zweimaliges Sonifizieren gelöst. Die anschließende Enzymreaktion erfolgt für 60 Minuten bei 37°C. Hierzu werden 45 µl der Substratlösung mit 1 µl Inhibitor entsprechender Konzentration (gelöst in DMSO, zur Kontrolle wird reine DMSO-Lösung verwendet) und 5 µl Enzymlösung (konditioniertes Medium) inkubiert. Im Anschluß werden 3 µl des Testansatzes auf eine HPTLC-Platte (Kieselgel 60, Merck) aufgetragen und der freigesetzte Fluoreszenzfarbstoff zum Nachweis mit einem Fließmittel (Diäthylether : Petroleumbenzin : Essigsäure [78:22:1]) getrennt. Nach dem Abdampfen des Fließmittels wird die Platte in einen Fluoreszenz-Scanner eingelesen. Als Maß für die Enzymaktivität ist eine gesteigerte Freisetzung des Fluoreszenzfarbstoffes in der ungehemmten Reaktion zu beobachten.

### 2.3. Auswertung der EL-Hemmwirkung:

In Abhängigkeit der verwendeten Inhibitorkonzentration ergibt sich eine Reduktion der enzymatischen Aktivität, die Inhibitiorkonzentration, bei welcher eine halbmaximale Enzymaktivität beobachtet wird, wird als IC₅ₒ bezeichnet.

### 2.4. Weiterer Test auf Hemmung der EL:

Zur Charakterisierung der enzymatischen Aktivität von endothelialer Lipase und der Wirkung von Inhibitoren wird das Phospholipase-spezifische Substrat 1,2-bis-(4,4-difluoro-5,7-dimethyl-4-bora-3a,4a-diaza-s-indacene-3-undecanoyl)-sn-glycero-3-phosphocholine, (Hersteller Molecular Probes) verwendet. Durch Hydrolyse der A1 Esterbindung dieses Phospholipids durch das Enzym wird der Fluoreszenzfarbstoff Bodipy freigesetzt, der nach Trennung durch Dünnschichtchromatographie auf einer HPTLC-Platte (Kieselgel 60, Merck) oder direkt im Reaktionsgefäß durch Messen der Fluoreszenz nachgewiesen werden kann.
Zur Herstellung der Substratlösung werden 100 µg 1,2-bis-(4,4-difluoro-5,7-dimethyl-4-bora-3a,4a-diaza-s-indacene-3-undecanoyl)-sn-glycero-3-phospho-choline (Hersteller Molecular Probes) in 100 µl DMSO gelöst und in 2,4 mg Tripalmitin (Sigma) in 393 µl Chloroform aufgenommen, welches 20mg/ ml DOP - Cholin (1,2-Dioleoyl-sn-glycero-3-phosphocholin) enthält. 39,3 µl dieses Lipidgemisches werden in ein frisches Reaktionsgefäß überführt und das Lösungsmittel abgedampft. Das Lipidgemisch wird in 4 ml 200 mM TRIS-HCl, 150 mM Natriumchlorid, pH = 7,4 durch zweimaliges Sonifizieren gelöst. Die anschließende Enzymreaktion erfolgt für 90 Minuten bei 37°C. Hierzu werden 20 µl der Substratlösung mit 2 µl Inhibitor entsprechender Konzentration (gelöst in 10% DMSO, zur Kontrolle wird 10%ige DMSO-Lösung verwendet) und 2 µl Enzymlösung (konditioniertes Medium) inkubiert. Im Anschluß werden 4 µl des Testansatzes auf eine HPTLC-Platte (Kieselgel 60, Merck) aufgetragen und der freigesetzte Fluoreszenzfarbstoff zum Nachweis mit einem Fließmittel (Diethylether : Petroleumbenzin : Essigsäure [78:22:1]) getrennt. Nach dem Abdampfen des Fließmittels wird die Platte in einen Fluoreszenz-Scanner eingelesen.
Als Maß für die Enzymaktivität ist eine gesteigerte Freisetzung des Fluoreszenzfarbstoffes in der ungehemmten Reaktion zu beobachten.

In diesem Test zeigten die Verbindungen der Beispiele folgende von IC₅₀ -Werte:

| Beispiel | IC₅₀ [µM] EL |
|---|---|
| 1 | 0.13 |
| 2 | 0.51 |
| 3 | 0.06 |
| 4 | 0.01 |
| 5 | 0.95 |
| 6 | 0,02 |
| 7 | 0,23 |

### Verfahren zur Herstellung

Die Herstellung der erfindungsgemäßen Verbindungen der allgemeinen Formel I erfolgt nach an und für sich bekannten Methoden z.B. durch Acylierung von substituierten, bzw. unsubstituiertem Benzooxazol-2-on-derivaten II mit Carbamoylchloriden III (Methode A), oder in zwei Stufen durch Umsetzung von Benzooxazol-2on-derivaten II mit Phosgen oder Äquivalenten wie Chlorcarbonsäuretrichlormethylester, Carbonsäureditrichlormethylester oder Chlorameisensäure-4-nitrophenylester und weiterer Umsetzung des erhaltenen Benzooxazol-2on-carbonsäurederivats mit Aminen IV (Methode B). Für Verbindungen bei denen R2 Wasserstoff ist, können die Benzooxazol-2-on-derivate II auch mit den entsprechenden Isocyanaten V R1-N=C=O umgesetzt werden.

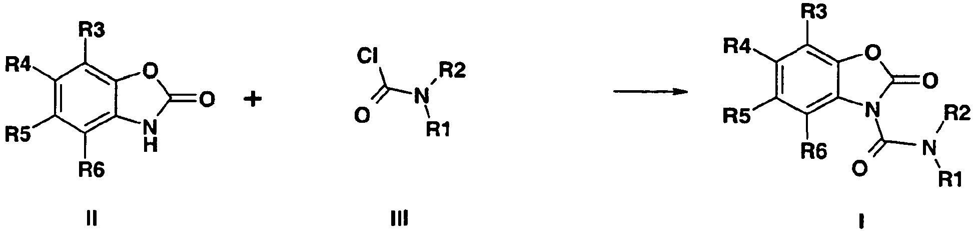

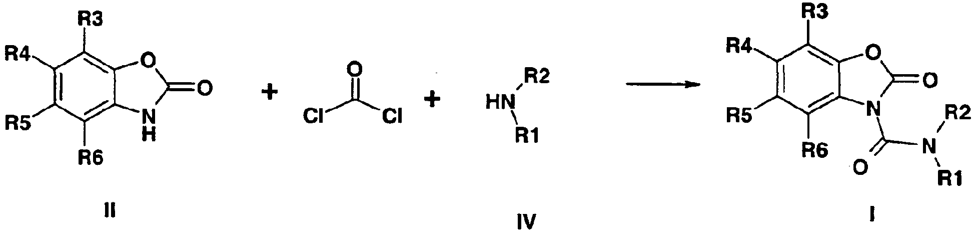

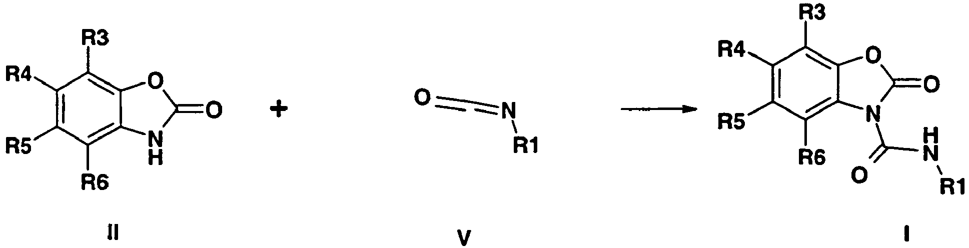

Da bei diesen Reaktionen in der Regel Säuren freigesetzt werden, empfiehlt es sich zur Beschleunigung Basen wie Pyridin, Triethylamin, Natronlauge oder Alkalicarbonate zu zusetzen. Die Reaktionen können in weiten Temperaturbereichen durchgeführt werden. In der Regel hat es sich als vorteilhaft herausgestellt, bei 0°C bis zum Siedepunkt des verwendeten Lösungsmittels zu arbeiten. Als Lösemittel kommen beispielsweise Methylenchlorid, THF, DMF, Toluol, Essigester, n-Heptan, Dioxan, Diethylether oder Pyridin zum Einsatz. Wenn unter wasserfreien Bedingungen gearbeitet wird, haben sich auch starke Basen wie Lithiumhydrid, Natriumhydrid oder Kalium-tert.-butylat in aprotischen Lösungsmitteln wie THF oder DMF bewährt.
Die als Ausgangsverbindungen II eingesetzten Benzooxazol-2-on-derivate sind kommerziell erhältlich oder lassen sich nach Literatur bekannten Verfahren herstellen (z.B. C. Flouzat, Y. Bresson, A. Mattio, J. Bonnet, G. Guillaumet J: Med.Chem 1993, 36, 497-503; F. Mutterer, C.D. Weis, J. Het. Chem. 1976, 13, 1103-1104;).

Die nachfolgend aufgeführten Beispiele dienen zur Erläuterung der Erfindung.

### Beispiele

### Beispiel 1:

### 6-Acetyl-2-oxo-benzooxazol-3-carbonsäurebenzylamid

100 mg (0.565 mmol) 6-Acetyl-3H-benzooxazol-2-on wurden in 5 ml Pyridin gelöst. Nach Zugabe von 90.3 mg (0.678 mmol) Benzylisocyanat wurde 5 h bei 80°C gerührt, nochmals die gleiche Menge Benzylisocyanat zugegeben und 5 h bei 100°C gerührt. Das Reaktionsgemisch wurde eingeengt und über präparative HPLC (PR18, Acetonitril/Wasser 0. 1 % TFA) gereinigt. Ausbeute: 27 mg (16 %), M+H+: 311.1.

### Beispiel 2:

### 6-Acetyl-2-oxo-benzooxazol-3-carbonsäure2-methyl-benzylamid

Analog Beispiel 1 wurden 100 mg (0.565 mmol) 6-Acetyl-3H-benzooxazol-2-on mit 99.8 mg (0.678 mmol) 2-Methyl-benzylisocyanat umgesetzt. Ausbeute: 63 mg (34 %), M+H+: 325.15.

### Beispiel 3:

### 6-Acetyl-2-oxo-benzooxazol-3-carbonsäurehexylamid

Analog Beispiel 1 wurden 100 mg (0.565 mmol) 6-Acetyl-3H-benzooxazol-2-on mit 86.2 mg (0.678 mmol) 1-Isocyanato-hexan umgesetzt. Ausbeute: 77 mg (45 %), M+H+: 305.15.

### Beispiel 4:

### 5-Chloro-2-oxo-benzooxazol-3-carbonsäurehexylamid

Analog Beispiel 1 wurden 100 mg (0.59 mmol) 5-Chloro-3H-benzooxazol-2-on mit 112.6 mg (0.885 mmol) 1-Isocyanato-hexan in Gegenwart von Ethyl-diisopropylamin in THF bei 70°C umgesetzt. Ausbeute: 125 mg (71 %), M+H+: 297.1.

### Beispiel 5:

### 2-Oxo-benzooxazol-3-carbonsäure2-methyl-benzylamid

Analog Beispiel 1 wurden 100 mg (0.74 mmol) 3H-Benzooxazol-2-on mit 130.7 mg (0.888 mmol) 1-Isocyanatomethyl-2-methyl-benzen in Dioxan bei 60°C umgesetzt. Ausbeute: 76 mg (36 %), M+H+: 283.25.

### Beispiel 6:

### 2-Oxo-benzooxazol-3-carbonsäurehexylamid

Analog Beispiel 1 wurden 100 mg (0.74 mmol) 3H-Benzooxazol-2-on mit 113 mg (0.888 mmol) 1-Isocyanato-hexan in Dioxan bei 80°C umgesetzt. Ausbeute: 163 mg (84 %), M+H+: 263.13.

### Beispiel 7:

### 5-Chloro-2-oxo-benzooxazol-3-carbonsäurebenzylamid

Analog Beispiel 1 wurden 100 mg (0.59 mmol) 5-Chloro-3H-benzooxazol-2-on mit 94 mg (0.7 mmol) Isocyanatomethyl-benzen in Dioxan bei 80°C umgesetzt. Ausbeute: 73 mg (41 %), M+H+: 303.05.

## Patentansprüche

1. Verbindung der allgemeinen Formel I 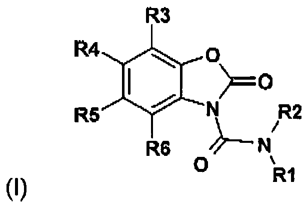 **dadurch gekennzeichnet, dass**
R1 (C₅-C₁₆)-Alkyl, (C₁-C₄)-Alkylen-(C₆-C₁₀)-Aryl, wobei Aryl durch Halogen, (C₁-C₆)-Alkyl, (C₁-C₃)-Alkyloxy, Hydroxy, (C₁-C₆)-Alkylmercapto, Amino, (C₁-C₆)-Alkylamino, di-(C₂-C₁₂)-Alkylamino, mono-(C₁-C₆)-Alkylaminocarbonyl, di-(C₂-C₈)-Alkylaminocarbonyl, (C₁-C₆)-Alkoxycarbonyl, (C₁-C₆)-Alkylcarbonyl, Cyano, Trifluormethyl, Trifluormethyloxy, (C₁-C₆)-Alkylsulfonyl, Aminosulfonyl ein oder mehrfach substituiert sein kann;
R2 Wasserstoff;
R3, R4, R5, R6 unabhängig voneinander Wasserstoff, Halogen, (C₁-C₆)-Alkyl, (C₁-C₃)-Alkyloxy-(C₁-C₃)-Alkylen, Hydroxy, (C₁-C₆)-Alkylmercapto, Amino, (C₁-C₆)-Alkylamino, di-(C₂-C₁₂)-Alkylamino, (C₁-C₆)-Alkylcarbonyl, COOR7, Trifluormethyl, (C₁-C₆)-Alkylsulfonyl, (C₁-C₆)-Alkylsulfinyl, Aminosulfonyl, Pentafluorsulfanyl, (C₆-C₁₀)-Aryl, (C₅-C₁₂)-Heteroaryl, CO-NR9R10, O-CO-NR9R10, O-CO-(C₁-C₆)-Alkylen-CO-O-(C₁-C₆)-Alkyl, O-CO-( C₁-C₆)-Alkylen-CO-OH, O-CO-(C₁-C₆)-Alkylen-CO-NR9R10 oder unsubstituiertes oder ein oder mehrfach durch F substituiertes (C₁-C₆)-Alkyloxy; mit der Maßgabe, dass höchstens einer der Substituenten R3, R4, R5 oder R6 Halogen ist; und mit der Maßgabe, dass höchstens einer der Substituenten R3, R4, R5 oder R6 (C₁-C₆)-Alkyl ist;
R7 Wasserstoff, (C₁-C₆)-Alkyl, Benzyl;
R9, R10 unabhängig voneinander Wasserstoff, (C₁-C₆)-Alkyl, -(C₆-C₁₀)-Aryl, (C₅-C₁₂)-Heteroaryl, (C₃-C₁₂)-Cycloalkyl, (C₁-C₄)-Alkylen-(C₆-C₁₀)-Aryl, (C₁-c₄)-Alkylen-(C₅-C₁₂)-Heteroaryl, (C₁-C₄)-Alkylen-(C₄-C₁₂)-Cycloalkyl, (C₈-C₁₄)-Bicyclus;
bedeuten,
die tautomeren Formen der Verbindungen sowie deren physiologisch verträgliche Salze.

2. Verbindung der Formel I gemäß Anspruch 1, **dadurch gekennzeichnet, dass**
R1 (C₆-C₁₂)-Alkyl, (C₁-C₃)-Alkylen-(C₆-C₁₀)-Aryl, wobei Aryl, durch Halogen, (C₁-C₆)-Alkyl, (C₁-C₃)-Alkyloxy, Hydroxy, Amino, (C₁-C₆)-Alkylamino, Trifluormethyl ein oder mehrfach substituiert sein kann;
R2 Wasserstoff;
R3, R4, R5, R6 unabhängig voneinander Wasserstoff, (C₁-C₃)-Alkyloxy-(C₁-C₃)-Alkylen, Hydroxy, COOR7, (C₁-C₆)-Alkylsulfonyl, (C₁-C₆)-Alkylsulfinyl, Aminosulfonyl, Pentafluorsulfanyl, (C₆-C₁₀)-Aryl, (C₅-C₁₂)-Heteroaryl, (C₁-C₆)-Alkylcarbonyl, CO-NR9R10, O-CO-NR9R10, O-CO-(C₁-C₆)-Alkylen -CO-O-(C₁-C₆)-Alkyl, O-CO-(C₁-C₆)-Alkylen-CO-OH, O-CO-(C₁-C₆)-Alkylen-CO-NR9R10;
R7 Wasserstoff, (C₁-C₆)-Alkyl, Benzyl;
R9, R10 unabhängig voneinander Wasserstoff, (C₁-C₆)-Alkyl, -(C₆-C₁₀)-Aryl, (C₁-C₄)-Alkylen-(C₆-C₁₀)-Aryl;
bedeuten.

3. Verbindung der Formel I gemäß Anspruch 1 oder 2, **dadurch gekennzeichnet, dass**
R1 (C₆-C₁₂)-Alkyl, Benzyl, wobei Benzyl durch Halogen, (C₁-C₆)-Alkyl, (C₁-C₃)-Alkyloxy, Hydroxy, Amino, (C₁-C₆)-Alkylamino, Trifluormethyl ein oder mehrfach substituiert sein kann;
R2 Wasserstoff;
R3, R4, R5, R6 unabhängig voneinander Wasserstoff, Hydroxy, COOR7, (C₁-C₆)-Alkylsulfonyl, Aminosulfonyl, Pentafluorsulfanyl, Phenyl, (C₁-C₆)-Alkylcarbonyl, CO-NR9R10, O-CO-NR9R10, O-CO-(C₁-C₆)-Alkylen - CO-O-(C₁-C₆)-Alkyl, O-CO-(C₁-C₆)-Alkylen-CO-OH, O-CO-(C₁-C₆)-Alkylen-CO-NR9R10;
R7 Wasserstoff, (C₁-C₆)-Alkyl;
R9, R10 unabhängig voneinander Wasserstoff, (C₁-C₆)-Alkyl;
bedeuten.

4. Verbindung der Formel I gemäß Ansprüchen 1, 2 oder 3 **dadurch gekennzeichnet, dass**
R3, R6 für Wasserstoff steht, R4 oder R5 nicht für Wasserstoff steht.

5. Verbindung der Formel I gemäß Ansprüchen 1 bis 4, **dadurch gekennzeichnet, dass**
R1 (C₆-C₁₂)-Alkyl, Benzyl, wobei Benzyl durch Halogen, (C₁-C₆)-Alkyl, (C₁-C₃)-Alkyloxy, Hydroxy, Amino, (C₁-C₆)-Alkylamino, Trifluormethyl ein oder mehrfach substituiert sein kann;
R2 Wasserstoff;
R3 Wasserstoff;
R4, R5 unabhängig voneinander Wasserstoff, Hydroxy, mono-(C₁-C₆)-Alkylaminocarbonyl, di-(C₂-C₈)-Alkylaminocarbonyl, COOR7, (C₁-C₆)-Alkylsulfonyl, Aminosulfonyl, Phenyl, (C₁-C₆)-Alkylcarbonyl, O-CO-NR9R10, O-CO-(C₁-C₆)-Alkylen -CO-O-(C₁-C₆)-Alkyl, O-CO-(C₁-C₆)-Alkylen-CO-OH, O-CO-(C₁-C₆)-Alkylen-CO-NR9R10;
R6 Wasserstoff;
bedeutet.

6. Verbindung der Formel I gemäß Ansprüchen 1 bis 5, **dadurch gekennzeichnet, dass**
R1 (C₆-C₁₂)-Alkyl, Benzyl, wobei Benzyl durch (C₁-C₆)-Alkyl ein oder mehrfach substituiert sein kann;
R2 Wasserstoff;
R3 Wasserstoff;
R4, R5 unabhängig voneinander Wasserstoff, Halogen oder (C₁-C₆)-Alkylcarbonyl;
R6 Wasserstoff;
bedeutet.

7. Arzneimittel enthaltend eine oder mehrere Verbindungen der Formel I gemäß den Ansprüchen 1 bis 6.

8. Verwendung der Verbindungen der Formel I gemäß den Ansprüchen 1 bis 6 zur Herstellung eines Medikaments zur Behandlung und/oder Prävention von Störungen des Fettsäurestoffwechsels und Glucoseverwertungsstörungen.

9. Verwendung der Verbindungen der Formel I gemäß den Ansprüchen 1 bis 6 zur Herstellung eines Medikaments zur Behandlung und/oder Prävention von Störungen, bei denen Insulin Resistenz eine Rolle spielt.

10. Verwendung der Verbindungen der Formel I gemäß den Ansprüchen 1 bis 6 zur Herstellung eines Medikaments zur Behandlung und/oder Prävention von Diabetes mellitus und der damit verbundenen Folgeerkrankungen.

11. Verwendung der Verbindungen der Formel I gemäß den Ansprüchen 1 bis 6 zur Herstellung eines Medikaments zur Behandlung und/oder Prävention von Dyslipidämien und deren Folgen.

12. Verwendung der Verbindungen der Formel I gemäß den Ansprüchen 1 bis 6 zur Herstellung eines Medikaments zur Behandlung und/oder Prävention von Zuständen, die mit dem Metabolischen Syndrom assoziert sind.

13. Verwendung der Verbindungen der Formel I gemäß den Ansprüchen 1 bis 6 zur Herstellung eines Medikaments zur Behandlung und/oder Prävention von Zuständen, die mit erniedrigtem HDL-Spiegel verbunden sind.

14. Verwendung der Verbindungen der Formel I gemäß den Ansprüchen 1 bis 6 zur Herstellung eines Medikaments zur Behandlung und/oder Prävention von atherosklerotischen Erkrankungen.

15. Verwendung der Verbindungen der Formel I gemäß den Ansprüchen 1 bis 6 in Kombination mit mindestens einem weiteren Wirkstoff zur Herstellung eines Medikaments zur Behandlung und/oder Prävention von Störungen, bei denen Insulin Resistenz eine Rolle spielt.

16. Verfahren zur Herstellung eines Arzneimittels enthaltend eine oder mehrere der Verbindungen der Formel I gemäß den Ansprüchen 1 bis 6, **dadurch gekennzeichnet, dass** diese mit einem pharmazeutisch geeigneten Träger vermischt wird und diese Mischung in eine für die Verabreichung geeignete Form gebracht wird.

17. Verfahren zur Herstellung von Verbindungen der allgemeinen Formel I gemäß den Ansprüchen 1 bis 6, **dadurch gekennzeichnet, dass** Benzooxazol-2-on-derivate der Formel II
a) mit Carbamoylchloriden der Formel III acyliert werden;
oder
b) in zwei Stufen zunächst mit Phosgen oder Äquivalenten wie Chlorcarbonsäuretrichlormethylester, Carbonsäureditrichlormethylester oder Chlorameisensäure-4-nitrophenylester und einem zweiten Schritt mit Aminen der Formel IV umgesetzt werden,
worin die Substituenten die oben genannten Bedeutungen haben.

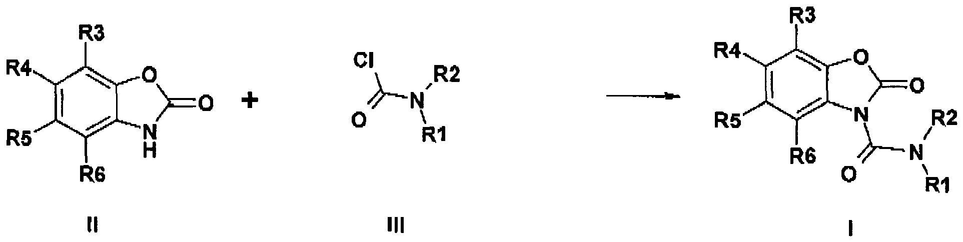

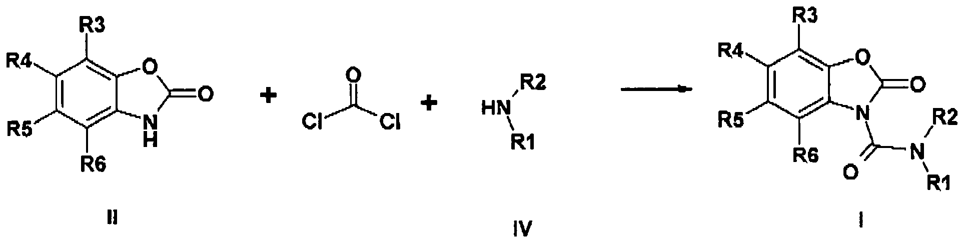

18. Verfahren zur Herstellung von Verbindungen der allgemeinen Formel I mit R2 Wasserstoff gemäß den Ansprüchen 1 bis 6, **dadurch gekennzeichnet, dass** Benzooxazol-2-on-derivate der Formel II mit Isocyanaten der Formel V: O=C=N-R1 umgesetzt werden.

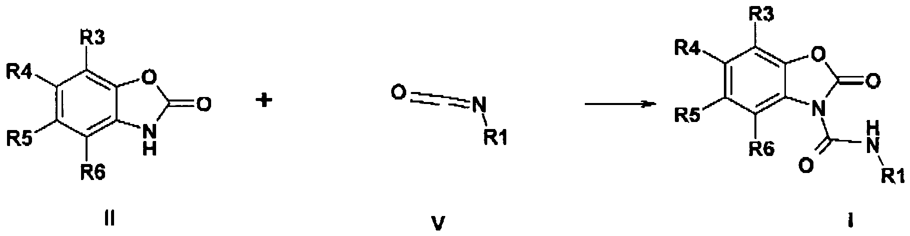

## Claims

1. A compound of the formula I 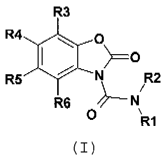 wherein the meanings are
R1 (C₅-C₁₆)-alkyl, (C₁-C₉)-alkylene-(C₆-C₁₀)-aryl, where aryl may be substituted one or more times by halogen, (C₁-C₆)-alkyl, (C₁-C₃)-alkyloxy, hydroxy, (C₁-C₆)-alkylmercapto, amino, (C₁-C₆)-alkylamino, di-(C₂-C₁₂)-alkylamino, mono-(C₁-C₆)-alkylaminocarbonyl, di-(C₂-C₈)-alkylaminocarbonyl, (C₁-C₆)-alkoxycarbonyl, (C₁-C₆)-alkylcarbonyl, cyano, trifluoromethyl, trifluoromethyloxy, (C₁-C₆)-alkylsulfonyl, aminosulfonyl;
R2 hydrogen;
R3, R4, R5, R6 independently of one another hydrogen, halogen, (C₁-C₈)-alkyl, (C₁-C₃)-alkyloxy-(C₁-C₃)-alkylene, hydroxy, (C₁-C₆)-alkylmercapto, amino, (C₁-C₆)-alkylamino, di-(C₂-C₁₂)-alkylamino, (C₁-C₆)-alkylcarbonyl, COOR7, trifluoromethyl, (C₁-C₆)-alkylsulfonyl, (C₁-C₆)-alkylsulfinyl, aminosulfonyl, pentafluorosulfanyl, (C₆-C₁₀)-aryl, (C₅-C₁₂)-heteroaryl, CO-NR9R10, O-CO-NR9R10, O-CO-(C₁-C₆)-alkylene-CO-O-(C₁-C₆)-alkyl, O-CO-(C₁-C₆) -alkylene-CO-OH, O-CO-(C₁-C₆)-alkylene-CO-NR9R10 or unsubstituted or mono- or poly-F-substituted (C₁-C₆)-alkyloxy; with the proviso that not more than one of the substituents R3, R4, R5 or R6 is halogen; and with the proviso that not more than one of the substituents R3, R4, R5 or R6 is (C₁-C₆)-alkyl;
R7 hydrogen, (C₁-C₆)-alkyl, benzyl;
R9, R10 independently of one another hydrogen, (C₁-C₆)-alkyl, -(C₆-C₁₀)-aryl, (C₅-C₁₂)-heteroaryl, (C₃-C₁₂)-cycloalkyl, (C₁-C₄)-alkylene-(C₆-C₁₀)-aryl, (C₁-C₄)-alkylene-(C₅-C₁₂)-heteroaryl, (C₁-C₄)-alkylene-(C₄-C₁₂)-cycloalkyl, (C₈-C₁₄)-bicycle;
the tautomeric forms of the compounds and the physiologically tolerated salts thereof.

2. A compound of the formula I as claimed in claim 1, wherein
R1 is (C₆-C₁₂)-alkyl, (C₁-C₃)-alkylene-(C₆-C₁₀)-aryl, where aryl may be substituted one or more times by halogen, (C₁-C₆)-alkyl, (C₁-C₃)-alkyloxy, hydroxy, amino, (C₁-C₆)-alkylamino, trifluoromethyl;
R2 is hydrogen;
R3, R4, R5, R6 are independently of one another hydrogen, (C₁-C₃)-alkyloxy-(C₁-C₃)-alkylene, hydroxy, COOR7, (C₁-C₆)-alkylsulfonyl, (C₁-C₆)-alkylsulfinyl, aminosulfonyl, pentafluorosulfanyl, (C₆-C₁₀)-aryl, (C₅-C₁₂)-heteroaryl, (C₁-C₆)-alkylcarbonyl, CO-NR9R10, O-CO-NR9R10, O-CO-(C₁-C₆)-alkylene-CO-O-(C₁-C₆)-alkyl, O-CO-(C₁-C₆)-alkylene-CO-OH, O-CO-(C₁-C₆)-alkylene-CO-NR9R10;
R7 is hydrogen, (C₁-C₆)-alkyl, benzyl;
R9, R10 are independently of one another hydrogen, (C₁-C₆) -alkyl, (C₆-C₁₀) -aryl, (C₁-C₄) -alkylene-(C₆-C₁₀)-aryl.

3. A compound of the formula I as claimed in claim 1 or 2, wherein
R1 is (C₆-C₁₂)-alkyl, benzyl, where benzyl may be substituted one or more times by halogen, (C₁-C₆)-alkyl, (C₁-C₃)-alkyloxy, hydroxy, amino, (C₁-C₆)-alkylamino, trifluoromethyl;
R2 is hydrogen;
R3, R4, R5, R6 are independently of one another hydrogen, hydroxy, COOR7, (C₁-C₆)-alkylsulfonyl, aminosulfonyl, pentafluorosulfanyl, phenyl, (C₁-C₆)-alkylcarbonyl, CO-NR9R10, O-CO-NR9R10, O-CO-(C₁-C₆)-alkylene-CO-O-(C₁-C₆)-alkyl, O-CO-(C₁-C₆)-alkylene-CO-OH, O-CO-(C₁-C₆)-alkylene-CO-NR9R10;
R7 is hydrogen, (C₁-C₆)-alkyl;
R9, R10 are independently of one another hydrogen, (C₁-C₆)-alkyl.

4. A compound of the formula I as claimed in claims 1, 2 or 3, wherein
R3, R6 is hydrogen, R4 or R5 is not hydrogen.

5. A compound of the formula I as claimed in claims 1 to 4, wherein
R1 is (C₆-C₁₂)-alkyl, benzyl, where benzyl may be substituted one or more times by halogen, (C₁-C₆)-alkyl, (C₁-C₃)-alkyloxy, hydroxy, amino, (C₁-C₆)-alkylamino, trifluoromethyl;
R2 is hydrogen;
R3 is hydrogen;
R4, R5 are independently of one another hydrogen, hydroxy, mono-(C₁-C₆)-alkylaminocarbonyl, di-(C₂-C₈)-alkylaminocarbonyl, COOR7, (C₁-C₆)-alkylsulfonyl, aminosulfonyl, phenyl, (C₁-C₆)-alkylcarbonyl, O-CO-NR9R10, O-CO-(C₁-C₆)-alkylene -CO-O-(C₁-C₆)-alkyl, O-CO-(C₁-C₆)-alkylene-CO-OH, O-CO-(C₁-C₆)-alkylene-CO-NR9R10;
R6 is hydrogen.

6. A compound of the formula I as claimed in claims 1 to 5, wherein
R1 is (C₆-C₁₂)-alkyl or benzyl, where benzyl may be substituted one or more times by (C₁-C₆)-alkyl;
R2 is hydrogen;
R3 is hydrogen;
R4, R5 are independently of one another hydrogen, halogen or (C₁-C₆)-alkylcarbonyl;
R6 is hydrogen.

7. A medicament comprising one or more compounds of the formula I as claimed in claims 1 to 6.

8. The use of the compounds of the formula I as claimed in claims 1 to 6 for producing a medicament for the treatment and/or prevention of disorders of fatty acid metabolism and glucose utilization disorders.

9. The use of the compounds of the formula I as claimed in claims 1 to 6 for producing a medicament for the treatment and/or prevention of disorders in which insulin resistance is involved.

10. The use of the compounds of the formula I as claimed in claims 1 to 6 for producing a medicament for the treatment and/or prevention of diabetes mellitus and the sequelae associated therewith.

11. The use of the compounds of the formula I as claimed in claims 1 to 6 for producing a medicament for the treatment and/or prevention of dyslipidemias and the sequelae thereof.

12. The use of the compounds of the formula I as claimed in claims 1 to 6 for producing a medicament for the treatment and/or prevention of conditions associated with the metabolic syndrome.

13. The use of the compounds of the formula I as claimed in claims 1 to 6 for producing a medicament for the treatment and/or prevention of conditions associated with reduced HDL level.

14. The use of the compounds of the formula I as claimed in claims 1 to 6 for producing a medicament for the treatment and/or prevention of atherosclerotic disorders.

15. The use of the compounds of the formula I as claimed in claims 1 to 6 in combination with at least one further active ingredient for producing a medicament for the treatment and/or prevention of disorders in which insulin resistance is involved.

16. A process for producing a medicament comprising one or more of the compounds of the formula I as claimed in claims 1 to 6, which comprises mixing the latter with a pharmaceutically suitable carrier and converting this mixture into a form suitable for administration.

17. A process for preparing compounds of the general formula I as claimed in claims 1 to 6, which comprises benzooxazol-2-one derivatives of the formula II
a) being acylated with carbamoyl chlorides of the formula III;
or
b) in two stages being reacted first with phosgene or equivalents such as trichloromethyl chlorocarbonate, ditrichloromethyl carbonate or 4-nitrophenyl chloroformate and in a second step with amines of the formula IV,
in which the substituents have the meanings indicated above.

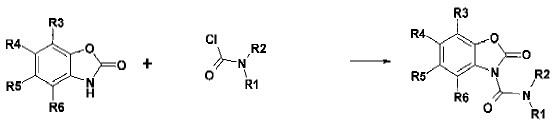

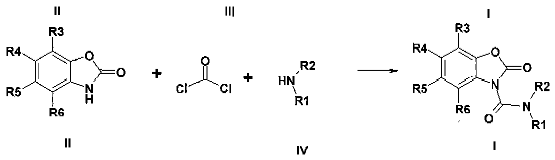

18. A process for preparing compounds of the general formula I with R2 hydrogen as claimed in claims 1 to 6, which comprises reacting benzooxazol-2-one derivatives of the formula II with isocyanates of the formula V: O=C=N-R1.

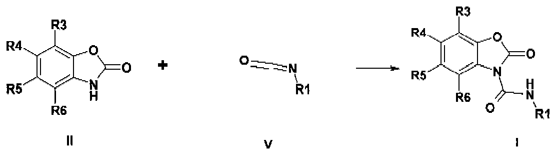

## Revendications

1. Composé de formule générale I 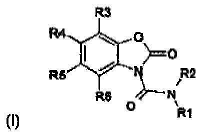 **caractérisé en ce que**
R1 signifie alkyle en (C₅-C₁₆), alkylène en (C₁-C₄)-aryle en (C₆-C₁₀), aryle pouvant être substitué une ou plusieurs fois par halogène, alkyle en (C₁-C₆), alkyloxy en (C₁-C₃), hydroxy, alkylmercapto en (C₁-C₆), amino, alkylamino en (C₁-C₆), di-alkylamino en (C₂-C₁₂), mono-alkylaminocarbonyle en (C₁-C₆), di-alkylaminocarbonyle en (C₂-C₈), alcoxycarbonyle en (C₁-C₆), alkylcarbonyle en (C₁-C₆), cyano, trifluorométhyle, trifluorométhyloxy, alkylsulfonyle en (C₁-C₆), aminosulfonyle ;
R2 signifie hydrogène ;
R3, R4, R5, R6 signifient indépendamment les uns des autres hydrogène, halogène, alkyle en (C₁-C₆), alkyloxy en (C₁-C₃)-alkylène en (C₁-C₃), hydroxy, alkylmercapto en (C₁-C₆), amino, alkylamino en (C₁-C₆), di-alkylamino en (C₂-C₁₂), alkylcarbonyle en (C₁-C₆), COOR7, trifluorométhyle, alkylsulfonyle en (C₁-C₆), alkylsulfinyle en (C₁-C₆), aminosulfonyle, pentafluorosulfanyle, aryle en (C₆-C₁₀), hétéroaryle en (C₅-C₁₂), CO-NR9R10, O-CO-NR9R10, O-CO-alkylène en (C₁-C₆)-CO-O-alkyle en (C₁-C₆), O-CO-alkylène en (C₁-C₆)-CO-OH, O-CO-alkylène en (C₁-C₆)-CO-NR9R10, ou alkyloxy en (C₁-C₆) non substitué ou substitué une ou plusieurs fois par F ;
à condition qu'au plus un des substituants R3, R4, R5 ou R6 soit un halogène ;
et à condition qu'au plus un des substituants R3, R4, R5 ou R6 soit un alkyle en (C₁-C₆) ;
R7 signifie hydrogène, alkyle en (C₁-C₆), benzyle ;
R9, R10 signifient indépendamment l'un de l'autre hydrogène, alkyle en (C₁-C₆), aryle en (C₆-C₁₀), hétéroaryle en (C₅-C₁₂), cycloalkyle en (C₃-C₁₂), alkylène en (C₁-C₄)-aryle en (C₆-C₁₀), alkylène en (C₁-C₄)-hétéroaryle en (C₅-C₁₂), alkylène en (C₁-C₄)-cycloalkyle en (C₄-C₁₂), bicycle en (C₈-C₁₄) ;
les formes tautomères des composés, ainsi que leurs sels physiologiquement compatibles.

2. Composé de formule I selon la revendication 1, **caractérisé en ce que**
R1 signifie alkyle en (C₆-C₁₂), alkylène en (C₁-C₃)-aryle en (C₆-C₁₀), aryle pouvant être substitué une ou plusieurs fois par halogène, alkyle en (C₁-C₆), alkyloxy en (C₁-C₃), hydroxy, amino, alkylamino en (C₁-C₆), trifluorométhyle ;
R2 signifie hydrogène ;
R3, R4, R5, R6 signifient indépendamment les uns des autres hydrogène, alkyloxy en (C₁-C₃)-alkylène en (C₁-C₃), hydroxy, COOR7, alkylsulfonyle en (C₁-C₆), alkylsulfinyle en (C₁-C₆), aminosulfonyle, pentafluorosulfanyle, aryle en (C₆-C₁₀), hétéroaryle en (C₅-C₁₂), alkylcarbonyle en (C₁-C₆), CO-NR9R10, O-CO-NR9R10, O-CO-alkylène en (C₁-C₆)-CO-O-alkyle en (C₁-C₆), O-CO-alkylène en (C₁-C₆)-CO-OH, O-CO-alkylène en (C₁-C₆)-CO-NR9R10 ;
R7 signifie hydrogène, alkyle en (C₁-C₆), benzyle ;
R9, R10 signifient indépendamment l'un de l'autre hydrogène, alkyle en (C₁-C₆), aryle en (C₆-C₁₀), alkylène en (C₁-C₄)-aryle en (C₆-C₁₀).

3. Composé de formule I selon la revendication 1 ou 2, **caractérisé en ce que**
R1 signifie alkyle en (C₁-C₁₂), benzyle, benzyle pouvant être substitué une ou plusieurs fois par halogène, alkyle en (C₁-C₆), alkyloxy en (C₁-C₃), hydroxy, amino, alkylamino en (C₁-C₆), trifluorométhyle ;
R2 signifie hydrogène ;
R3, R4, R5, R6 signifient indépendamment les uns des autres hydrogène, hydroxy, COOR7, alkylsulfonyle en (C₁-C₆), aminosulfonyle, pentafluorosulfanyle, phényle, alkylcarbonyle en (C₁-C₆), CO-NR9R10, O-CO-NR9R10, O-CO-alkylène en (C₁-C₆)-CO-O-alkyle en (C₁-C₆), O-CO-alkyléne en (C₁-C₆)-CO-OH, O-CO-alkylène en (C₁-C₆)-CO-NR9R10 ;
R7 signifie hydrogène, alkyle en (C₁-C₆) ;
R9, R10 signifient indépendamment l'un de l'autre hydrogène, alkyle en (C₁-C₆).

4. Composé de formule I selon les revendications 1, 2 ou 3, **caractérisé en ce que**
R3, R6 représentent hydrogène, R4 ou R5 ne représente pas hydrogène.

5. Composé de formule I selon les revendications 1 à 4, **caractérisé en ce que**
R1 signifie alkyle en (C₆-C₁₂), benzyle, benzyle pouvant être substitué une ou plusieurs fois par halogène, alkyle en (C₁-C₆), alkyloxy en (C₁-C₃), hydroxy, amino, alkylamino en (C₁-C₆), trifluorométhyle ;
R2 signifie hydrogène ;
R3 signifie hydrogène ;
R4, R5 signifient indépendamment l'un de l'autre hydrogène, hydroxy, mono-alkylaminocarbonyle en (C₁-C₆), di-alkylaminocarbonyle en (C₂-C₈), COOR7, alkylsulfonyle en (C₁-C₆), aminosulfonyle, phényle, alkylcarbonyle en (C₁-C₆), O-CO-NR9R10, O-CO-alkylène en (C₁-C₆)-CO-O-alkyle en (C₁-C₆), O-CO-alkylène en (C₁-C₆)-CO-OH, O-CO-alkylène en (C₁-C₆)-CO-NR9R10 ;
R6 signifie hydrogène.

6. Composé de formule I selon les revendications 1 à 5, **caractérisé en ce que**
R1 signifie alkyle en (C₆-C₁₂), benzyle, benzyle pouvant être substitué une ou plusieurs fois par alkyle en (C₁-C₆) ;
R2 signifie hydrogène ;
R3 signifie hydrogène ;
R4, R5 signifient indépendamment l'un de l'autre hydrogène, halogène ou alkylcarbonyle en (C₁-C₆) ;
R6 signifie hydrogène.

7. Médicament contenant un ou plusieurs composés de formule I selon les revendications 1 à 6.

8. Utilisation des composés de formule I selon les revendications 1 à 6 pour la fabrication d'un médicament pour le traitement et/ou la prévention des troubles du métabolisme des acides gras et des troubles de l'utilisation du glucose.

9. Utilisation des composés de formule I selon les revendications 1 à 6 pour la fabrication d'un médicament pour le traitement et/ou la prévention des troubles dans lesquels la résistance à l'insuline joue un rôle.

10. Utilisation des composés de formule I selon les revendications 1 à 6 pour la fabrication d'un médicament pour le traitement et/ou la prévention du diabète sucré et des complications associées.

11. Utilisation des composés de formule I selon les revendications 1 à 6 pour la fabrication d'un médicament pour le traitement et/ou la prévention des dyslipidémies et leurs conséquences.

12. Utilisation des composés de formule I selon les revendications 1 à 6 pour la fabrication d'un médicament pour le traitement et/ou la prévention des états associés avec le syndrome métabolique.

13. Utilisation des composés de formule I selon les revendications 1 à 6 pour la fabrication d'un médicament pour le traitement et/ou la prévention des états associés avec un niveau d'HDL abaissé.

14. Utilisation des composés de formule I selon les revendications 1 à 6 pour la fabrication d'un médicament pour le traitement et/ou la prévention des maladies athérosclérotiques.

15. Utilisation des composés de formule I selon les revendications 1 à 6 en combinaison avec au moins un agent actif supplémentaire pour la fabrication d'un médicament pour le traitement et/ou la prévention des troubles dans lesquels la résistance à l'insuline joue un rôle.

16. Procédé de fabrication d'un médicament contenant un ou plusieurs des composés de formule I selon les revendications 1 à 6, **caractérisé en ce que** ceux-ci sont mélangés avec un véhicule pharmaceutiquement approprié et ce mélange est mis sous une forme appropriée pour l'administration.

17. Procédé de fabrication de composés de formule générale I selon les revendications 1 à 6, **caractérisé en ce que** des dérivés de benzooxazol-2-one de formule II
a) sont acylés avec des chlorures de carbamoyle de formule III ; ou
b) sont mis en réaction en deux étapes, tout d'abord avec du phosgène ou des équivalents tels que l'ester trichlorométhylique d'acide chlorocarboxyllique, l'ester ditrichlorométhylique d'acide carboxylique ou l'ester 4-nitrophénylique d'acide chloroformique et, lors d'une seconde étape, avec des amines de formule IV les substituants ayant les significations données précédemment.

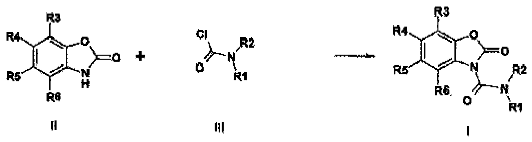

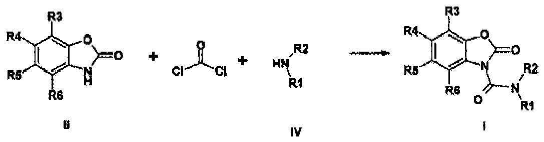

18. Procédé de fabrication de composés de formule générale I avec R2 hydrogène selon les revendications 1 à 6, **caractérisé en ce que** des dérivés de benzooxazol-2-one de formule II sont mis en réaction avec des isocyanates de formule V : O=C=N-R1.
